## Europäisches Patentamt
## European Patent Office
## Office européen des brevets

(19)

(11) Numéro de publication: **0 320 398**
**A2**

# (12) DEMANDE DE BREVET EUROPEEN

(21) Numéro de dépôt: **88403131.1**

(22) Date de dépôt: **09.12.88**

(51) Int. Cl.⁴: **C 12 P 19/04**
C 08 B 37/00, C 13 K 13/00
//(C12P19/04,C12R1:22)

(30) Priorité: **11.12.87 FR 8717285**

(43) Date de publication de la demande:
**14.06.89 Bulletin 89/24**

(84) Etats contractants désignés:
**AT BE CH DE ES FR GB GR IT LI LU NL SE**

(71) Demandeur: **BIOEUROPE**
**4 Impasse Didier Daurat**
**F-31400 Toulouse (FR)**

(72) Inventeur: **Graber-Gubert, Marianne Geneviève Pierre**
**5 rue P. Féral**
**F-31000 Toulouse (FR)**

**Morin, Joseph Charles André**
**11 rue Maran**
**F-31400 Toulouse (FR)**

**Duchiron, Francis Lucien**
**4 rés. La Fontaine-aux-Bois 25 rue J. Mermoz**
**F-77210 Avon (FR)**

**Monsan, Pierre Frédéric**
**Renoufail, Mondonville**
**F-31700 Blagnac (FR)**

(74) Mandataire: **Colas, Jean-Pierre et al**
**Cabinet de Boisse 37, avenue Franklin D. Roosevelt**
**F-75008 Paris (FR)**

Le (Les) microorganisme(s) a (ont) été déposé(s) auprès CNCM sous le(s) numéro(s) I-714.

(54) **Culture d'un nouveau micro-organisme du genre Klebsiella Sp., et procédé de production d'un mélange d'oses à teneur élevée en rhamnose mettant en oeuvre cette culture.**

(57) L'invention se rapporte aux biotechnologies.

Elle concerne notamment des cultures de micro-organisme Klebsiella sp. présentant les caractéristiques de la souche BEC 441 déposée sous le No. I-714 à la Collection Nationale de Cultures de Micro-organismes de l'Institut Pasteur, ainsi que de souches obtenues à partir de la souche BEC 441 par mutation, ces souches étant capables de produire des polysaccharides riches en rhamnose par fermentation dans un milieu nutritif contenant des sources assimilables de carbone et d'azote, et des substances minérales.

Application à la production d'un mélange d'oses à forte teneur en rhamnose.

EP 0 320 398 A2

## Description

### Culture d'un nouveau micro-organisme du genre Klebsiella sp., et procédé de production d'un mélange d'oses à teneur élevée en rhamnose mettant en oeuvre cette culture.

L'invention concerne une culture d'un nouveau micro-organisme du genre Klebsiella sp. ou de mutants de ce dernier, et un procédé de production d'un mélange d'oses à teneur élevée en rhamnose mettant en oeuvre cette culture.

Il est connu que certains micro-organismes du genre Klebsiella peuvent produire, dans certaines conditions de croissance, des polysaccharides, notamment des exopolysaccharides, en une proportion de l'ordre de quelques dixièmes de gramme par litre de milieu de croissance, le rhamnose pouvant constituer jusqu'à 66% de ces exopolysaccharides. Voir à ce sujet l'article de B.A. Bryan, R.J. Linhardt et L. Daniels paru dans la revue "Applied and Environmental Microbiology", vol. 51, N° 6, pages 1304-1308 (Juin 1986). Toutefois, le rendement en rhamnose produit est trop faible pour donner lieu à une application industrielle.

La Demanderesse a maintenant trouvé qu'il était possible de produire du rhamnose avec des rendements beaucoup plus élevés que ceux rapportés dans l'article ci-dessus en ayant recours à une culture d'un nouveau micro-organisme du genre Klebsiella sp.

La présente invention a donc pour premier objet de fournir une culture d'un nouveau micro-organisme du genre Klebsiella sp. capable de produire du rhamnose avec un rendement élevé.

Un deuxième objet de l'invention est de fournir des cultures de mutants sélectionnés dudit micro-organisme capables de produire du rhamnose avec un rendement encore amélioré.

Un troisième objet de l'invention est de fournir un procédé de production d'un mélange d'oses contenant du rhamnose.

Plus précisément, l'invention concerne une culture d'un nouveau micro-organisme Klebsiella sp., présentant les caractéristiques de la souche BEC 441 déposée sous le numéro I-714 en date du 10 Novembre 1987 à la Collection Nationale de Cultures de Micro-organismes (C.N.C.M.) de l'Institut Pasteur, 25, rue de Docteur Roux - 75724 PARIS CEDEX 15. et capable de produire des polysaccharides contenant du rhamnose, en grande partie sous forme d'exopolysaccharides, par fermentation dans un milieu nutritif contenant des sources assimilables de carbone, d'azote et de substances minérales.

Le nouveau micro-organisme qui a été isolé à partir de boues d'une station d'épuration, présente les caractéristiques suivantes :

ELEMENTS DE BASE
- BACILLES DE FORME HOMOGENE, DE GRAND DIAMETRE, SANS ARRANGEMENT NOTABLE
- MOBILITE -
- COLORATION DE GRAM⁻
- LYSE PAR KOH à 3% : + + +
- CULTURE APPARENTE EN GELOSE PROFONDE : AERO-ANAEROBIE

- CULTURE EN BOUILLON, NaCl 0% : + + +
- CULTURE EN BOUILLON, NaCl 2% : + + +
- CULTURE EN BOUILLON, NaCl 4% : + + +
- CULTURE EN BOUILLON, NaCl 6% : + +
- CULTURE EN BOUILLON, NaCl 8% : +
- CULTURE EN BOUILLON, NaCl 10% : -
[MILIEU DE BASE : PYG (peptone : 10 g/l, extrait de levure : 3 g/l, glucose : 1g/l)]
- CULTURE à 10°C : + + +
- CULTURE à 20°C : + + +
- CULTURE à 41°C : + + +
- CULTURE à 45°C : -
[MILIEU DE BASE : BHI (cerveau, infusion de coeur, diagnostic Pasteur)]
- CULTURE RAPIDE ET LUXURIANTE SUR GELOSES ORDINAIRES :
COLONIES ETENDUES, LISSES, CHARNUES, BLANCHATRES, D'UN DIAMETRE DE 8-10 MM (GELOSE COLUMBIA, 30°C, AIR, 4 JOURS D'INCUBATION
- CULTURE PROTOTROPHE
- PRODUCTION ACIDE A PARTIR DU GLUCOSE : + + +
- PRODUCTION DE GAZ A PARTIR DU GLUCOSE :
à 10°C : + + + t
à 30°C : + + +
à 41°C : -
- REACTION DE VOGES -PROSKAUER : + + +
- NITRATE REDUCTASE : + + +
- NITRITE REDUCTASE : +
- THIOSULFATE REDUCTASE : -
- TETRATHIONATE REDUCTASE : -
- UREASE : + + +
- PRODUCTION D'INDOLE : -
- PHENYLALANINE DESAMINASE : -
-TRYPTOPHANE DESAMINASE : -
- "ARGININE DIHYDROLASE" : -
- "ORNITHINE DECARBOXYLASE" -
- "LYSINE DECARBOXYLASE" : -
- γ-GLUTAMYL - TRANSFERASE : + + +
- HYDROLYSE DE L'oNPb-GALACTOSIDE : + + +
- HYDROLYSE DU pNPb-XYLOSIDE : -
- HYDROLYSE DU pNPb-GLUCURONATE : -
- HYDROLYSE DE L'AMIDON INSOLUBLE : +
- HYDROLYSE DU TWEEN 80 : -
- LIPASE SUR GELOSE AU JAUNE D'OEUF: -
- DNASE EXTRACELLULAIRE : -
- GELATINASE (FRAZIER) : -
- CASEINASE : -
- ACIDIFICATION EN API® 50 CH (vendue par API System S.A., La Balme-les-Grottes, 38390 Montalieu-Vercieu, France)
MILIEU DE SUSPENSION : MTL (milieu M63 ci-dessous + peptone 5 g/l + extrait de levure 0,5 g/l)
INDICATEUR : ROUGE DE PHENOL
LECTURES : JOUR, 1, 5, 9

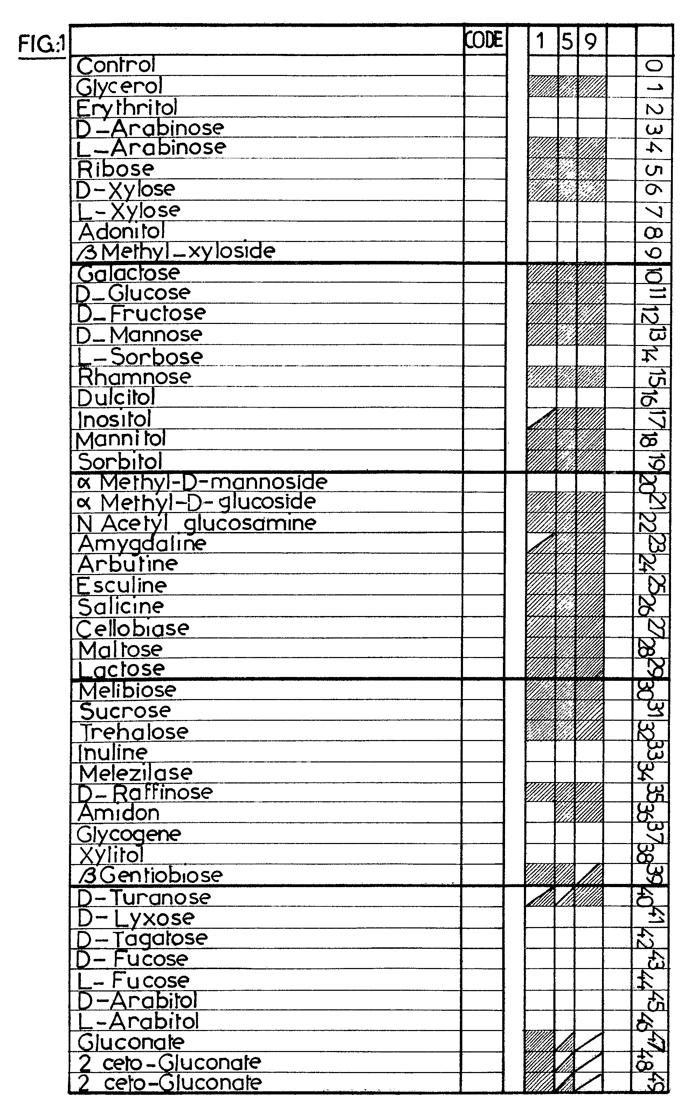

Les résultats sont récapitulés sur la figure 1. - CULTURES EN MILIEU M63 (milieu minéral pour l'étude des sources de carbone, dont la composition est :

KH$_2$PO$_4$ : 3,9 g/l
K$_2$HPO$_4$ : 11,2 g/l
(NH$_4$)$_2$SO$_4$ : 2 g/l
MgSO$_4$,7H$_2$O : 0,2 g/l
FeSO$_4$,7H$_2$O : 0,05 g/l
EN PRESENCE DE
D-GLUCOSE : + + +
Ac. ACETIQUE : + + +
Ac. MALONIQUE : -
Ac. CITRIQUE : + + +
Ac. HYDROXY-3-BENZOIQUE : -
L-HYDROXY-PROLINE : -
REACTIONS A 30°C AIR

EXPLICATIONS DES SYMBOLES :
 - : REACTION OU CULTURE NEGATIVE
 f : FAIBLE
 t : TARDIVE
 + : POSITIVE
 + + : TRES POSITIVE
 + + + : PARTICULIEREMENT INTENSE

ELEMENTS CHIMIOTAXONOMIQUES

COMPOSITION EN LIPIDES POLAIRES

Analyse comparative par chromatographie sur couche mince de silice
Procédé d'extraction : chloroforme/méthanol 1:2
chromatographie monodimensionnelle :
solvant de migration : chloroforme/méthanol/eau/ac.acétique

65:25:4:0,8

| révélation des phospholipides | : molybdate d'ammonium |
|---|---|
| lipides aminés | : ninhydrine |
| glycolipides | : α-naphtol |
| lipides totaux | : vanilline/H$_2$SO$_4$ |

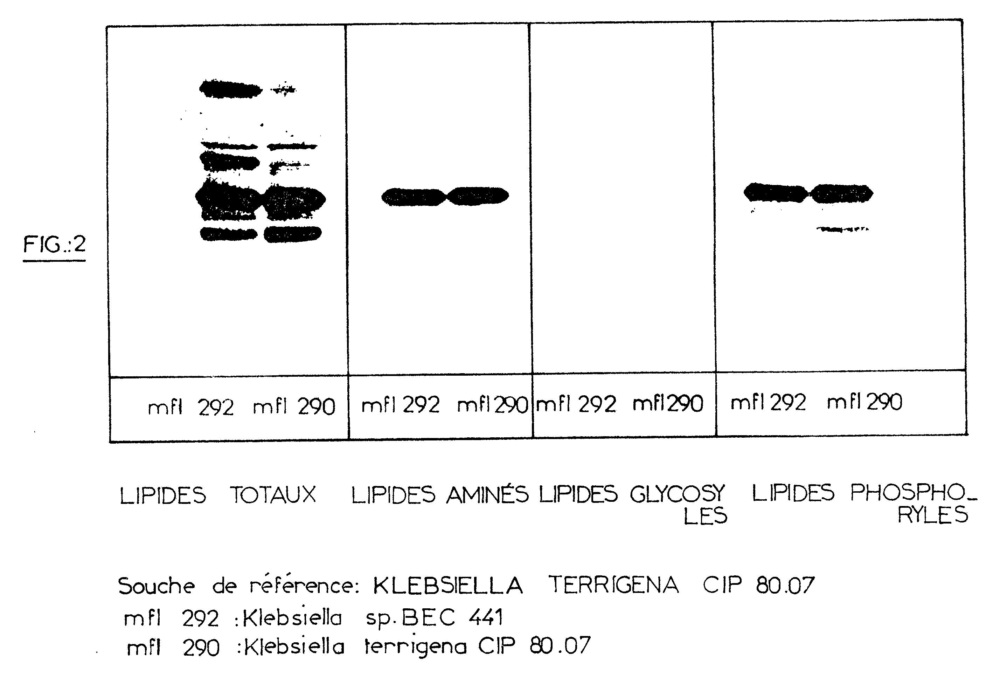

souche de référence :mfl 290 : KLEBSIELLA TERRIGENA CIP 80.07
Les chromatogrammes obtenus sont reproduits à la figure 2.

La souche Klebsiella sp. I. 714 donne une réaction positive à la réaction de Neufeld (gonflement de la capsule) lorsqu'elle est testée contre les séra anticapsulaires K14 et K44. Elle donne une réaction négative contre plusieurs autres séra anticapsulaires dont les séra K1 à K13, K15, K17 à K43, K46, K47, K50, K51, K54, K55, K59 à K62, K64 à K67, et K70 à K72. La souche I.714 donne une réaction de Neufeld faiblement positive contre les séra K16, K45, K48, K49, K52, K53, K56 à K58, K63, K68 et K69. La souche I.714 s'apparente donc aux sérotypes K14 et K44.

La présente invention concerne également les cultures de mutants du micro-organisme Klebsiella sp. BEC 441. Des mutants utiles peuvent être obtenus par simple sélection de mutants spontanés isolés à partir d'une culture précédente en fermenteur, ou bien en soumettant une culture de Klebsiella sp. BEC 441 à l'action d'un rayonnement énergétique (rayons U.V., rayons X par exemple) ou d'un agent chimique [par exemple l'ozone, l'acide nitreux, la NTG(N-méthyl-N'-nitro-N-nitrosoguanidine) ou l'EMS (méthane-sulfonate d'éthyle] en vue de tuer une portion importante des micro-organismes, en cultivant les micro-organismes survivants et en sélectionnant ceux produisant davantage de polysaccharides.

La présente invention concerne, en outre, un procédé d'obtention d'un mélange d'oses à teneur élevée en rhamnose qui consiste à faire fermenter une culture de Klebsiella sp. BEC 441 ou de mutants sélectionnés de celle-ci dans un milieu nutritif comprenant une source de carbone, une source d'azote et des sels minéraux appropriés, à un pH d'environ 6 à 8, à une température d'environ 30 à 35°C, sous aération et agitation, pendant 2 à 12 jours, à isoler les polysaccharides résultants du milieu de fermentation, puis à hydrolyser ces polysaccharides et à ajuster le pH de l'hydrolysat résultant dans la gamme de 5 à 9 environ.

L'invention concerne aussi le rhamnose produit à l'aide d'une culture selon l'invention.

Comme source de carbone, on peut utiliser, de façon non limitative, des substances telles que le sorbitol, le mannitol, le glucose, le glycérol et leurs mélanges. On préfère, à ce jour, le sorbitol. Il n'est pas indispensable d'utiliser une source de carbone chimiquement pure. On peut, par exemple, utiliser du sorbitol de qualité technique contenant, entre autres, du mannitol comme impuretés.

A titre indicatif, on peut utiliser de 5 à 100g de source de carbone par litre de milieu nutritif, de préférence 10 à 50g/l.

Comme source d'azote, on peut utiliser, de façon non limitative, des substances telles que la peptone de caséine (obtenue par hydrolyse acide ou enzymatique de la caséine), la liqueur de trempage du maïs, l'extrait de levure, la peptone de soja, la farine de pomme de terre, la L-phénylalanine, la thréonine, le sulfate d'ammonium et leurs mélanges. A ce jour, on préfère la peptone de caséine, et la liqueur de trempage du maïs et la farine de pomme de terre. La L-phénylalanine donne aussi de bons résultats mais a l'inconvénient d'être relativement coûteuse.

A titre indicatif, on peut utiliser de 0,5 à 10g de source d'azote par litre de milieu nutritif, de préférence 1 à 5g/l.

Un paramètre important est le rapport en poids carbone/azote du milieu nutritif. Il est avantageux d'utiliser un rapport d'au moins 5 et pouvant aller jusqu'à 100 et plus.

Comme sels minéraux, il faut inclure un sel de magnésium, par exemple MgSO$_4$, et au moins un sel phosphate, par exemple choisi parmi M$_2$HPO$_4$ où M est un métal alcalin ou l'ion ammonium. Le sel de magnésium peut être présent, par exemple, à raison de 0,1 à 0,3g/l, tandis que le sel phosphate peut être présent, par exemple, à raison de 2 à 15g/l. Il est avantageux d'utiliser un mélange de M$_2$HPO$_4$ et de MH$_2$PO$_4$ car un tel mélange permet de régler le pH du milieu nutritif et, en outre, sert de tampon.

La température du milieu de fermentation peut aller de 30 à 35°C et est de préférence de 32-33°C.

Le pH du milieu doit être compris entre 6 et 8 et est de préférence voisin de la neutralité (pH 7).

Les conditions d'aération doivent être suffisantes pour maintenir la pression partielle d'oxygène au-dessus du milieu de fermentation. En général une aération de l'ordre de 0,3 à 3 VVM (volume d'air/volume de milieu/minute) sera suffisante.

Il faut aussi maintenir le milieu de fermentation sous bonne agitation pour éviter un épaississement excessif de celui-ci. En général, une agitation à une vitesse de 300 à 1500 tours/minute conviendra.

Il est avantageux par ailleurs, quoique non indispensable, d'incorporer au milieu nutritif un agent tensio-actif non toxique pour le micro-organisme afin de faciliter la séparation des polysaccharides du milieu de fermentation. Un exemple d'agent tensio-actif utile est l'agent tensio-actif non ionique vendu dans le commerce sous la marque déposée "Tween 80" (mono-oléate de polyoxyéthylène-sorbitol). On peut en incorporer, par exemple de 0,5 à 2g/l, de préférence environ 1g/litre.

Il est également avantageux d'incorporer au milieu nutritif une petite quantité d'extrait de levure (si on ne l'utilise pas comme source d'azote principale) afin de réaliser un apport de vitamines.

La durée de la fermentation sera en général de l'ordre de 2 à 12 jours, selon les conditions opératoires et l'objectif de concentration en polysaccharides à obtenir.

L'isolement des polysaccharides produits qui sont sous la forme d'exopolysaccharides et de polysaccharides liés aux cellules, peut être réalisé, par exemple, en soumettant le milieu de fermentation à un traitement thermique à 70-120°C environ pendant 10 minutes à 1 heure environ (pour extraire la fraction des polysaccharides liés aux cellules), puis en centrifugeant à froid et en recueillant la couche surnageante claire. Cette couche contient la quasi-totalité des polysaccharides. On peut alors, si désiré, précipiter les polysaccharides de la couche surnageante recueillie par addition d'un liquide organique non-solvant tel que l'acétone ou un alcool inférieur tel que l'éthanol ou le propanol, puis séparer par filtration ou centrifugation le précipité et finalement le sécher. En variante, la couche surnageante peut être lyophilisée.

Les polysaccharides produits par Klebsiella sp. BEC 441 (I. 714) sont constitués d'une unité répétée hexasaccharide. Cet hexasaccharide comporte du rhamnose, du galactose et de l'acide glucuronique dans des rapports molaires de 3:2:1, respectivement. Il comporte une chaîne latérale ramifiée en position 3 sur le galactose. Au point de branchement de cette chaîne latérale se trouve directement rattaché l'acide uronique qui fait donc partie de la chaîne latérale. Cette dernière est terminée par un résidu de rhamnose.

On procède ensuite à une hydrolyse des polysaccharides au moyen d'un acide fort tel que $H_2SO_4$ OU HCl. pour convertir les polysaccharides en oses, puis à un réglage du pH de l'hydrolysat par une base, telle que NaOH ou KOH, afin d'obtenir un pH dans la gamme de 5 à 9 environ, de préférence voisin de la neutralité.

L'hydrolyse peut être conduite directement sur la couche surnageante claire ou sur le produit séché ou lyophilisé. L'hydrolyse peut être effectuée par exemple à l'aide d'$H_2SO_4$ à 100°C pendant 6 heures, la concentration finale en acide dans le volume traité étant d'environ 2N.

Typiquement l'hydrolysat neutralisé a la composition d'oses suivante :

| | | |
|---|---|---|
| rhamnose | 64 - 80 | (pourcentage en rapport molaire) |
| glucose | 2 - 22 | (pourcentage en rapport molaire) |
| galactose | 12 - 21 | (pourcentage en rapport molaire) |
| acide glucuronique | 0 - 12 | (pourcentage en poids) |

Si on le désire le rhamnose peut être isolé des autres oses par des techniques connues de séparation d'oses. A titre indicatif, les hydrolysats neutralisés peuvent être soumis une séparation par chromatographie sur colonne échangeuse d'ions après les avoir dessalés et remis dans un tampon approprié à la résine échangeuse d'ions utilisée. Un exemple de résine convenable est la Dowex 1x4 avec emploi d'un tampon borate, mais bien d'autres résines sont utilisables.

Le mélange d'oses à teneur élevée en rhamnose ou le rhamnose isolé de ce mélange est un produit de départ utile pour la synthèse d'agents de saveur ou arômes tel que le furanéol et ses dérivés.

Par ailleurs, les polysaccharides produits par la culture de l'invention ont les utilités des polysaccharides en général et peuvent être utilisés, par exemple, comme stabilisant, agents de suspension, dispersant, épaississant, agent filmogène, agent de rétention d'eau, coagulant, colloïde, lubrifiant, par exemple, dans des lessives, textiles, adhésifs, papier, peintures, et aliments, ainsi que pour la récupération assistée du pétrole.

Afin d'illustrer la présente invention, on donne ci-après les exemples non limitatifs suivants.

Dans chacun de ces exemples, on a employé un inoculum de la culture de l'invention ayant une densité optique de 0,15 à 600 nm en une proportion de 10 millilitres/litre de milieu de fermentation.

EXEMPLE 1

On a placé dans un fermenteur de laboratoire (fermenteur, modèle 105500, vendu par la Société BIOLAFITTE, Saint-Germain en Laye, France, d'une capacité de 2 litres) un milieu de fermentation ayant la composition suivante :

| sorbitol | : | 20 | g/l |
|---|---|---|---|
| phénylalanine | : | 1 | g/l |
| MgSO$_4$.7H$_2$O | : | 0,2 | g/l |
| K$_2$HPO$_4$ | : | 9 | g/l |
| KH$_2$PO$_4$ | : | 3 | g/l |
| Tween ® 80 | : | 1 | g/l |

extrait de levure de boulangerie : 20 mg/l   A ce milieu on a ajouté l'inoculum spécifié précédemment et on a effectué une fermentation dans les conditions suivantes :

| Température | : | 30°C |
|---|---|---|
| Aération | : | 0,5 VVM |
| Agitation | : | 400 à 600 tours/mn |
| pH initial | : | 7 (non régulé). |

Après 7 jours de fermentation, la teneur en rhamnose était de 3 g/l de milieu de fermentation, comme déterminée par spectrophotométrie. On a alors ajouté au milieu 50 g/l de sorbitol et 5 g/l de phénylalanine. Quatre jours plus tard la teneur en rhamnose était de 5,5 g/l.

EXEMPLE 2

On a effectué une fermentation dans les mêmes équipements qu'à l'exemple 1, mais en utilisant le milieu de fermentation suivant :

| sorbitol | : | 20 | g/l |
|---|---|---|---|
| phénylalanine | : | 2 | g/l |
| tween ® 80 | : | 1 | g/l |
| MgSO$_4$.7H$_2$O | : | 0,2 | g/l |
| K$_2$HPO$_4$ | : | 9 | g/l |
| KH$_2$PO$_4$ | : | 3 | g/l |
| Extrait de levure | : | 20 | mg/l |

Les conditions de fermentation étaient les mêmes qu'à l'exemple 1.

Au bout de 8 jours de fermentation, on a obtenu une teneur en rhamnose de 5,5 g/litre de milieu de fermentation.

EXEMPLE 3

On a répété l'exemple 2 si ce n'est que les proportions de sorbitol et de phénylalanine ont été portées à 50 g/l et 5 g/l, respectivement.

On a pu ainsi obtenir, au bout de 7 jours de fermentation seulement, une teneur en rhamnose de 5,5 g/litre de milieu de fermentation.

EXEMPLE 4

Cet exemple décrit la préparation de souches mutantes hyperproductrices de polysaccharides riches en rhamnose de la souche de départ BEC 441, par un traitement aux rayons U.V.

Une culture de la souche BEC 441 est réalisée sur le milieu suivant :

| - sorbitol | : | 10 | g/l |
|---|---|---|---|
| - phénylalanine | : | 1 | g/l |
| - tween ® 80 | : | 1 | g/l |
| - MgSO$_4$7H$_2$O | : | 0,2 | g/l |
| - K$_2$HPO$_4$ | : | 9 | g/l |
| - KH$_2$PO$_4$ | : | 3 | g/l |

Après 4 jours d'incubation à 30°C sous aération (0,5 VVM), 5ml de cette culture sont centrifugés (5 mn à 4000 t/mn), et le culot est repris dans 5 ml d'eau physiologique.

La suspension cellulaire ainsi obtenue est introduite dans une boîte de Pétri et placée sous une lampe U.V. (Philips 5W) à 20 cm de distance. Pendant toute la durée de l'exposition, une agitation manuelle est effectuée. Après 1 mn d'exposition, les cellules survivantes (environ 1% des cellules totales) sont réintroduites dans 20 ml du milieu précédemment décrit et mises à incuber pendant 3 heures à 30°C, afin de réaliser une expression de phénotype.

Après cette incubation, les cellules sont étalées en boîte de Pétri sur le milieu suivant :

| - sorbitol | : | 50 | g/l |
|---|---|---|---|
| - phénylalanine | : | 5 | g/l |
| - tween ® 80 | : | 1 | g/l |
| - MgSO$_4$,7H$_2$O | : | 0,2 | g/l |
| - K$_2$HPO$_4$ | : | 9 | g/l |
| - KH$_2$PO$_4$ | : | 3 | g/l |
| - Agar | : | 15 | g/l |

Après une semaine d'incubation à 30°C, les colonies mutantes ayant produit davantage de polysaccharides sont identifiées par la présence d'une zone mucoïde autour de la colonie plus importante que sur les colonies non mutantes.

Ces colonies sont isolées, puis multipliées de la même manière que la souche initiale, et conservées.

EXEMPLE 5

Cet exemple décrit la préparation de souches mutantes hyperproductrices de polysaccharies riches en rhamnose de la souche de départ BEC 441, par un traitement à l'ozone.

Une culture de 1 litre est réalisée dans un fermenteur sur le milieu liquide et dans les conditions décrites à l'exemple 4.

Après 4 jours d'incubation, un traitement à l'ozone est effectué à l'aide d'un ozoneur de laboratoire. Pour cela, l'air entrant dans le fermenteur est dérivé par l'ozoneur avant d'être introduit dans le fermenteur. Après 1 heure de traitement, l'aération est rétablie.

Le nombre de cellules viables est déterminé avant et après le traitement, le taux de survivants après le traitement est d'environ 10%.

Une expression de phénotype est réalisée en laissant les cellules incubées 3 heures supplémentaires après le traitement. Puis les cellules sont étalées sur boîte de Pétri pour la recherche de mutants hyperproducteurs ; cette recherche est réalisée de la même manière qu'à l'exemple 4.

EXEMPLE 6

Cet exemple illustre la production de polysaccharides riches en rhamnose à l'aide de souches mutantes.

Identification des souches utilisées :

- EP et EP 2 :

sont obtenues à partir de la souche BEC 441 par mutagénèse U.V. comme décrit à l'exemple 4.

- F₄ 1 et F₄ 2 :

sont des mutants spontanés isolés à partir d'une culture précédente en fermenteur. Les colonies mutantes spontanées hyperproductrices de polysaccharides riches en rhamnose sont identifiables par la présence d'une zone mucoïde entourant la colonie plus importante que sur les colonies non mutantes.

Milieu de culture employé :
- Sorbitol : 50 g/l
- Peptone de caséine : 4,5 g/l
- Phénylalanine : 0,1 g/l
- Extrait de levure : 0,05 g/l
- Tween ③ 80 : 1 g/l
- $MgSO_4.7H_2O$ : 0,2 g/l
- $K_2H\ PO_4$ : 1,8 g/l
- $KH_2PO_4$ : 0,6 g/l

Conditions de culture en fermenteur :
- Température : 32°C
- pH : régulé à 7,0 avec NaOH 1N
- Aération : 0,5 VVM
- Agitation : 400 à 1000 tours/minute

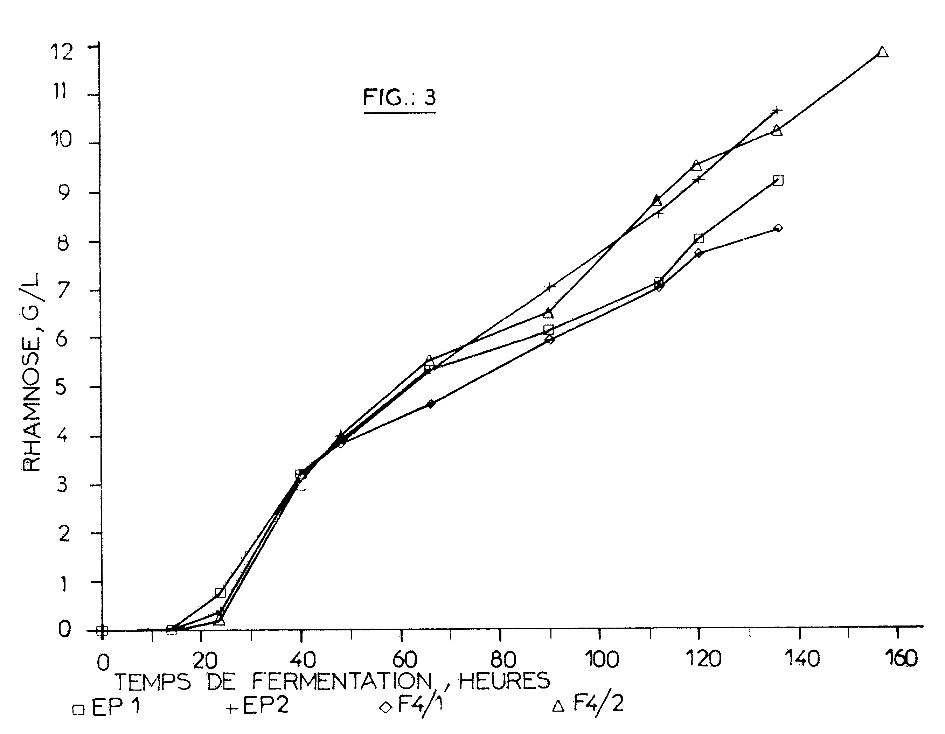

On suit jour après jour la production de rhamnose et on trace des courbes de la production de rhamnose en fonction de la durée de fermentation pour chacune des souches mutantes utilisées. Les courbes obtenues sont représentées sur la figure 3.

On voit que l'on peut obtenir jusqu'à 12 g de rhamnose par litre de milieu de fermentation après moins de 7 jours de fermentation.

**Revendications**

1. Cultures de micro-organisme Klebsiella sp. présentant les caractéristiques de la souche BEC 441 déposée sous le No. I-714 à la Collection Nationale de Cultures de Micro-organismes de l'Institut Pasteur, ainsi que de souches obtenues à partir de la souche BEC 441 par mutation, ces souches étant capables de produire des polysaccharides riches en rhamnose par fermentation dans un milieu nutritif contenant des sources assimilables de carbone et d'azote, et des substances minérales.

2. Procédé d'obtention d'un mélange d'oses à teneur élevée en rhamnose qui consiste à faire fermenter une culture de Klebsiella sp. BEC 441 ou de mutants sélectionnés de celle-ci dans un milieu nutritif comprenant une source de carbone, une source d'azote et des sels minéraux appropriés, à un pH d'environ 6 à 8, à une température d'environ 30 à 35°C, sous aération et agitation, pendant 2 à 12 jours, à isoler les polysaccharides résultants du milieu de fermentation, puis à hydrolyser ces polysaccharides et à ajuster le pH de l'hydrolysat résultant dans la gamme de 5 à 9 environ.

3. Procédé selon la revendication 2, caractérisé en ce que la source de carbone est choisie parmi le sorbitol, le mannitol, le glucose et le glycérol et est présente à raison de 5 à 100 g/litre de milieu nutritif.

4. Procédé selon la revendication 2 ou 3, caractérisé en ce que la source d'azote est choisie parmi la peptone de caséine, la liqueur de trempage du maïs, l'extrait de levure, la peptone de soja, la farine de pomme de terre, la L-phénylalanine, la thréonine et le sulfate d'ammonium, et est présente à raison de 0,5 à 10 g/litre de milieu nutritif.

5. Procédé selon l'une quelconque des revendications 2 à 4, caractérisé en ce que le rapport en poids carbone/azote du milieu nutritif est d'au moins 5.

6. Procédé selon l'une quelconque des revendications 2 à 5, caractérisé en ce que le milieu nutritif contient, en outre, des sels minéraux choisis parmi les sels de magnésium, les sels phosphate et leurs mélanges.

7. Procédé selon l'une quelconque des revendications 2 à 6, caractérisé en ce que le milieu nutritif contient en outre une petite quantité d'extrait de levure.

8. Rhamnose produit à l'aide d'une culture telle que définie à la revendication 1.

FIG.1

| | CODE | 1 | 5 | 9 | | |
|---|---|---|---|---|---|---|
| Control | | | | | | 0 |
| Glycerol | | | | | | 1 |
| Erythritol | | | | | | 2 |
| D—Arabinose | | | | | | 3 |
| L—Arabinose | | | | | | 4 |
| Ribose | | | | | | 5 |
| D—Xylose | | | | | | 6 |
| L—Xylose | | | | | | 7 |
| Adonitol | | | | | | 8 |
| β Methyl—xyloside | | | | | | 9 |
| Galactose | | | | | | 10 |
| D—Glucose | | | | | | 11 |
| D—Fructose | | | | | | 12 |
| D—Mannose | | | | | | 13 |
| L—Sorbose | | | | | | 14 |
| Rhamnose | | | | | | 15 |
| Dulcitol | | | | | | 16 |
| Inositol | | | | | | 17 |
| Mannitol | | | | | | 18 |
| Sorbitol | | | | | | 19 |
| α Methyl-D-mannoside | | | | | | 20 |
| α Methyl-D-glucoside | | | | | | 21 |
| N Acetyl glucosamine | | | | | | 22 |
| Amygdaline | | | | | | 23 |
| Arbutine | | | | | | 24 |
| Esculine | | | | | | 25 |
| Salicine | | | | | | 26 |
| Cellobiase | | | | | | 27 |
| Maltose | | | | | | 28 |
| Lactose | | | | | | 29 |
| Melibiose | | | | | | 30 |
| Sucrose | | | | | | 31 |
| Trehalose | | | | | | 32 |
| Inuline | | | | | | 33 |
| Melezilase | | | | | | 34 |
| D—Raffinose | | | | | | 35 |
| Amidon | | | | | | 36 |
| Glycogene | | | | | | 37 |
| Xylitol | | | | | | 38 |
| β Gentiobiose | | | | | | 39 |
| D—Turanose | | | | | | 40 |
| D—Lyxose | | | | | | 41 |
| D—Tagatose | | | | | | 42 |
| D—Fucose | | | | | | 43 |
| L—Fucose | | | | | | 44 |
| D—Arabitol | | | | | | 45 |
| L—Arabitol | | | | | | 46 |
| Gluconate | | | | | | 47 |
| 2 ceto-Gluconate | | | | | | 48 |
| 2 ceto-Gluconate | | | | | | 49 |

FIG.:2

| mfl 292 mfl 290 | mfl 292 mfl 290 | mfl 292 mfl 290 | mfl 292 mfl 290 |
|---|---|---|---|
| LIPIDES TOTAUX | LIPIDES AMINÉS | LIPIDES GLYCOSYLES | LIPIDES PHOSPHORYLES |

Souche de référence: KLEBSIELLA TERRIGENA CIP 80.07

mfl 292 : Klebsiella sp. BEC 441

mfl 290 : Klebsiella terrigena CIP 80.07

EP 0 320 398 A2

FIG.: 3

EP 0 320 398 A2